(19) Europäisches Patentamt
European Patent Office
Office européen des brevets

(11) **EP 4 472 591 B1**

(12) **EUROPEAN PATENT SPECIFICATION**

(45) Date of publication and mention
of the grant of the patent:
**22.07.2026  Bulletin 2026/30**

(21) Application number: **23703849.2**

(22) Date of filing: **27.01.2023**

(51) International Patent Classification (IPC):
*A61F 11/08* (2006.01)   *A61F 11/14* (2006.01)
*H04R 1/10* (2026.01)   *A61F 11/12* (2006.01)
*G10K 11/162* (2006.01)   *G10K 15/00* (2006.01)

(52) Cooperative Patent Classification (CPC):
**A61F 11/08; A61F 11/145; H04R 1/1083;**
A61F 11/12; G10K 11/162; G10K 15/00;
H04R 2460/15

(86) International application number:
**PCT/GB2023/050194**

(87) International publication number:
**WO 2023/144558 (03.08.2023 Gazette 2023/31)**

(54) **METHOD FOR HEARING PROTECTION DEVICES**

VERFAHREN FÜR GEHÖRSCHUTZVORRICHTUNGEN

PROCEDE POUR DISPOSITIFS DE PROTECTION AUDITIVE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **31.01.2022  GB 202201235**
**01.12.2022  GB 202218096**

(43) Date of publication of application:
**11.12.2024  Bulletin 2024/50**

(73) Proprietor: **Minuendo AS**
**0153 Oslo (NO)**

(72) Inventors:
• **KVALØY, Olav**
**0153 Oslo (NO)**
• **TRONES, Tom Alexander**
**0153 Oslo (NO)**

(74) Representative: **Dehns**
**10 Old Bailey**
**London EC4M 7NG (GB)**

(56) References cited:
WO-A1-2019/000089     WO-A1-2021/074651
US-A1- 2015 010 158     US-A1- 2021 241 747

**Description**

BACKGROUND OF THE INVENTION

This invention relates to hearing protection, in particular determining whether a hearing protection device is fitted and operating effectively.

In a variety of industries, for example construction and performance arts, employees are subjected to high amplitude noises. When exposure to high amplitude noises occurs over an extended period of time, it can result in permanent damage to the function of the ear i.e. to an individual's hearing. As such, appropriate protection against these high amplitude noises is important to reduce the health risks of working in such environments. Protection is required to limit an individual's exposure to both shorter periods of high amplitude noises, and noisy environments over a longer period of time (i.e. over a working shift).

Whilst protection, in the form of earplugs (both active and passive) and over-the-ear defenders, is available to reduce the health risk associated with exposure to high amplitude noises, this protection may be incorrectly fitted or not worn as appropriate. This may be because individuals find such protection uncomfortable or inconvenient to wear or have a flawed understanding of the level of risk they face in their current environment. Incorrectly fitted protection may result in inadequate attenuation or no attenuation being provided by the protection equipment. Whilst it is known that this is a problem, given that it is dependent on the compliance of many different individuals, it can be difficult to determine the extent of the problem.

Determining whether hearing protection has been worn over an extended period of time where an individual has been exposed to noise gives a valuable insight into compliance. This can be utilised to work towards minimising hearing damage in individuals.

US 2021/0241747 A1 describes a signal processing device for on ear detection of headsets.

SUMMARY OF THE INVENTION

When viewed from a first aspect, the invention provides a method of determining an effective state of an acoustic barrier as claimed in claim 1.

Thus it will be seen that, in accordance with embodiments of the invention, it can be determined whether an acoustic barrier is effective, and therefore whether a wearable hearing protection device is being used, from sound data which is collected continually during normal use of the hearing protection device. This facilitates monitoring of an individual's compliance with use of the wearable hearing protection device without requiring specific action or triggers.

Determining whether the wearable hearing protection device is being used could inform the provision of alerts if sound levels are deemed to be too loud in the surrounding environment and the wearable hearing protection device is detected as not in use. This may incentivise an individual to use their wearable hearing protection device, improve the fit of the device, or even fit supplementary hearing protection in order to further improve the attenuation of sound. It also facilitates appropriate intervention when protection is not properly being used. This may result in a reduced risk of damage to an individual's hearing.

Retrospectively identifying periods of time in collected sound data where the hearing protection is or isn't used makes it possible to monitor compliance over an extended period of time, e.g. over the course of a shift.

Furthermore, knowledge of whether the wearable hearing protection device is currently being used or not allows tailored data processing of the received sound data. For example, different processing steps may take into account the acoustic characteristics of the microphone's environment. This could include applying a filter to counteract resonance in the ear canal when receiving sound from a microphone on a fitted ear plug.

The received sound signals could comprise any measured sound characteristic, but preferably comprise a characteristic which is a measure of how loud the sound is, e.g. a sound pressure level (SPL).

In a set of embodiments, the signal representative of sound inside of the acoustic barrier (the "internal" sound) is received from a microphone positioned on a wearable hearing protection device designed for insertion into an ear canal. An example of a device for insertion into an ear canal is an earplug. In such devices the acoustic barrier is typically formed in the outer part of the ear canal. The device may be correctly fitted when the device is fully inserted into the ear canal. However, if the device in not fully inserted, the level of attenuation it provides will typically be reduced.

In another set of embodiments, the internal sound signal is received from a wearable hearing protection device comprising a pair of over-the-ear defenders in which the acoustic barrier is formed by providing a seal against the user's head around their respective outer ears. The wearable hearing protection device may comprise multiple hearing protection components. For example, for very noisy environments the hearing protection device may comprise a device for insertion into an ear canal and a pair of over-the-ear defenders. In such a system the signal representative of sound outside of the acoustic barrier (the "ambient" sound signal) should be measured outside the outermost acoustic barrier, and the signal representative of sound behind the acoustic barrier (the "internal" sound signal) should be measured behind the innermost acoustic barrier.

In embodiments in which the wearable hearing protection device comprises a pair of over-the-ear defenders, the device may be correctly fitted when the over-the-ear defenders are positioned to bear against the user's head and enclose their outer ears the respective acoustic barriers. Again however, if the device is not properly fitted, e.g. where the headband is incorrectly adjusted or a part of the outer ear is trapped beneath the cushioned seal such that the cushioned seal does not seal properly against the user's head, the level of attenuation provided will be reduced.

Whilst the microphone should be located acoustically behind the acoustic barrier provided by the wearable hearing protection device in order to measure sound behind the acoustic barrier, it will be appreciated that in practice it may be convenient to provide the microphone within the structure which forms the acoustic barrier. In such arrangements a channel can be provided from the microphone to behind the acoustic barrier.

In a set of embodiments, the ambient sound signal is received from a second microphone positioned on a wearable collar connected to an earpiece of the wearable hearing protection device.

In another set of embodiments, the ambient sound signal is received from a second microphone positioned on an earpiece of the wearable hearing protection device designed for insertion into an ear canal. Whilst the second microphone should be located acoustically outside the acoustic barrier provided by the wearable hearing protection device in order to measure sound outside the acoustic barrier, it will be appreciated that in practice it may be convenient to provide the second microphone within the structure which forms the acoustic barrier. In such arrangements a channel can be provided between the second microphone and the outside of the acoustic barrier. The second microphone could be located on a common printed circuit board (PCB) with the first microphone.

In another set of embodiments, the ambient sound signal is received from a second microphone positioned on a device provided separately to the wearable hearing protection device, e.g. by another device carried by the user such as a mobile device or smart phone.

The second microphone is used to measure the sound outside of the acoustic barrier in the local environment, e.g. the sound pressure levels an individual would be subjected to without wearing the hearing protection device.

The received sound signals are subsequently processed by applying the first filter, the second filter, and one or more further filters. Typically, each filter has a respective frequency characteristic. Each filter is applied to the internal sound signal received from behind the sound barrier, and applied to the ambient audio signal received from outside of it, to generate respective filtered internal and ambient sound signals. In a set of embodiments, each of the ambient sound signal and the internal sound signal are processed using five different filters, i.e a third, fourth and fifth filtered signal is generated for the internal sound signal and ambient sound signal respectively.

Applying a plurality of different filters to each of the internal sound signal and ambient sound signal respectively allows the frequency responses across a plurality of different frequency bands to be compared. The Applicant has appreciated that comparing the properties of the internal and ambient sound signals at a plurality of different frequencies is particularly advantageous for accurately determining the effective state of the hearing protection device. In particular, the Applicant has found that the shape of the frequency response of the internal sound signal is different to the shape of the ambient frequency response when the hearing protection device is fitted, on account of the differing acoustic environments. Accordingly, comparing the properties of a filtered internal sound signal and filtered ambient sound signal for several frequency bands may advantageously allow the differences in the shape of the response to be efficiently determined.

Each filter may have a different frequency characteristic, for example, different low-pass, bandpass and high-pass filters may be used. Any number of low-pass filters, bandpass filters and high-pass filters may be applied to the sound signals. In embodiments where one or more band-pass filters are used, each band-pass filter may have a different centre frequency. In embodiments where one or more high-pass filters or low-pass filters are used, each high-pass and/or low-pass filter may have a different cut-off frequency. Applying each filter to a received sound signal generates a filtered sound signal, isolated for a corresponding frequency band, wherein the frequency band is at least in part determined by the corresponding centre or cut-off frequency.

In a set of embodiments where five different filters are used, the filters comprise one low-pass filter, three band-pass filters, and one high-pass filter. Accordingly, a filtered internal sound signal and an ambient sound signal are generated for each of five different frequency bands.

For each filtered sound signal, an intensity value is calculated which represents the intensity of the sound in the corresponding frequency band. Accordingly, a set of ambient intensity values and a set of internal intensity values are generated for the corresponding received internal sound signal and received ambient sound signal respectively. The intensity values may correspond to a measure of the total or average sound energy within the corresponding frequency band, or may correspond to a measure of magnitude of the filtered signal at the centre frequency of the frequency band, for example.

Each of the intensity values in the set of internal intensity values is normalised by an internal intensity value at a selected normalisation frequency, to generate a normalised set of internal intensity values. Equivalently, each of the intensity values in the set of ambient intensity values is normalised by the magnitude of an ambient intensity value at the same selected normalisation frequency to generate a normalised set of ambient intensity values. In a set of embodiments, the selected normalisation frequency corresponds to the centre frequency of one of the filters used to filter the received signal. Alternatively, a normalisation frequency may be selected which has not already been used to filter the received signal. Normalising both the internal and ambient intensity values at a given point may advantageously facilitate comparing the shape of the internal sound signal and ambient sound signal across the range of frequency bands used, by eliminating any underlying systematic difference in levels.

In a set of embodiments, for each frequency band, the corresponding ambient intensity value and the internal intensity value are compared to calculate a comparison value. Accordingly, a set of comparison values may be generated, wherein each comparison value in the set corresponds to a respective frequency band. In embodiments where normalisation is carried out at a selected normalisation frequency, the normalised intensity values are compared to calculate the comparison value.

The comparison value may be any numerical measure which represents a difference between the internal and ambient intensity values. For example, the comparison value may be a ratio, or a difference. In some embodiments where the comparison value is a ratio, the ratio may be calculated by dividing the ambient intensity value by the internal intensity value. In some embodiments where the comparison value is a difference, the difference may be calculated by subtracting the internal intensity value from the ambient intensity value. In a set of embodiments, the comparison value is calculated as the squared difference between the ambient intensity value and the internal intensity value for the given frequency band.

The comparison value gives an indication of whether there is a difference between the acoustic response at the internal microphone, and the acoustic response at the external, ambient microphone for each frequency band. Accordingly, the comparison values can be used to indicate whether the acoustic barrier is effective, i.e., is fitted in the ear of the user. In embodiments where a squared difference, difference, or ratio as described above is used to calculate the comparison value for each frequency band, if the comparison value is large and positive, it indicates that the filtered ambient sound level outside of the hearing protection device is significantly different to the filtered internal sound level measured behind the acoustic barrier. A large and positive comparison value may therefore indicate that the hearing protection device is fitted, and accordingly indicate the corresponding effective state.

In a set of embodiments, for each set of comparison values that are generated, two or more comparison values in the set are combined to determine an effective state of the acoustic barrier- i.e. whether or not the barrier is in place and effective or is either not in place or not effective. The Applicant has appreciated that combining a plurality of comparison values across several different frequency bands effectively allows the shape of the frequency response of the received signals to be analysed.

In a set of embodiments, for each set of comparison values that are generated, two or more comparison values in the set are combined to determine an effectiveness metric, and the effectiveness metric is used to determine the effective state of the acoustic barrier.

In a set of embodiments, an optionally weighted summation is used to combine two or more comparison values to calculate an effectiveness metric, and the effectiveness metric is used to determine the effective state. A weight of one may be used for each value included in the summation, although weights may take other values. Using a summation means the results of the comparisons to determine the acoustic response at each filtered frequency band contribute directly to the overall determination of the effective state. In embodiments where the comparison value is calculated as the squared difference between the internal intensity value and ambient intensity value for example, a large and positive weighted summation may indicate that the hearing protection device is effectively fitted because there is a significant difference in the shape of the internal and ambient frequency responses.

In a set of embodiments, an effectiveness metric is calculated for each set of comparison values. In some embodiments, each calculated effectiveness metric is compared to a threshold value to determine a corresponding effective state. However, the Applicant has appreciated that determining an effective state for each effectiveness metric that is calculated can result in too much instantaneous variation of the determined effective state.

Accordingly, the effective state of the hearing protection device may be determined in dependence on the number of instances that the effectiveness metric exceeds the threshold, for a set of effectiveness metrics calculated over a period of time (e.g. for a set of effectiveness metrics calculated over a minute, if an effectives metric is calculated every second). The effective state of the hearing protection device may be determined as "In Ear" if a predetermined proportion, e.g. over a half, of the effectiveness metrics in the set of effectiveness metrics exceed the threshold value.

In some other embodiments, rather than comparing each effectiveness metric which is calculated to a threshold value, an average effectiveness metric is calculated for a set of effectiveness metrics calculated over a period of time, and the average effectiveness metric is compared to the threshold value.

For example, an effectiveness metric may be calculated every second, but the comparison to the threshold and consequent determination of the effective state may be made every minute. The average effectiveness metric may be any numerical average of the set of effectiveness metrics, e.g. a mean, median, or mode.

The effective state may be determined through any statistical analysis of a set of effectiveness metrics over time.

In a set of embodiments, the first filter is a band-pass filter, preferably having a centre frequency in the range 800-1200Hz. The first filter is applied to the internal sound signal received from behind the sound barrier, and to the ambient audio signal received from outside of it.

The first filtered ambient sound signal and the first filtered internal sound signal may be compared to calculate an attenuation value. The attenuation value may be a ratio, preferably dividing a measure of magnitude of the first filtered ambient sound signal by a measure of magnitude of the first filtered internal sound signal. The measure of magnitude may be a mean-square of the respective filtered signals. The mean-square values could be calculated before or after filtering.

The attenuation value gives an indication of whether the acoustic barrier is providing effective attenuation from the ambient sound in the surrounding environment. In examples using the ratio calculation described above, if the attenuation value is large and positive, it indicates that the sound level outside of the hearing protection device is greater than the internal sound level measured behind the acoustic barrier. A large and positive attenuation value therefore indicates effective attenuation.

In a set of embodiments, the second filter is a band-pass filter, preferably having a centre frequency in the range 150-500Hz. The second filter is applied to the ambient sound signal received from behind the acoustic barrier, and to the internal sound signal received from outside of it.

The second filtered ambient sound signal and second filtered internal sound signal may be compared to calculate an occlusion value. The occlusion value may be a ratio, preferably dividing the magnitude of the second filtered internal sound by the magnitude of the second filtered ambient sound signal. Calculating the occlusion value may provide greater accuracy for determining an indication of whether the acoustic barrier is effectively fitted in circumstances where there is a sound source behind the acoustic barrier, for example, when the individual wearing the hearing protection device is talking. At frequencies typically found when people are talking, the occlusion effect means that an effectively fitted acoustic barrier will result in a sound pressure level behind the acoustic barrier which is higher than outside the barrier. If the occlusion value is large and positive, it therefore suggests the presence of the occlusion effect. Therefore, a large and positive occlusion value indicates an effective acoustic barrier which will provide attenuation from external sounds.

The first and second comparisons may be combined, and used to determine the effective state of the acoustic barrier- i.e. whether or not the barrier is in place and effective or is either not in place or not effective.

An optionally weighted summation may be used to combine the attenuation value with the occlusion value to calculate an effectiveness metric. Preferably, a weight of one is used for each value included in the summation. Using a weighted summation means the results of the comparisons to determine behaviour of sound at the centre frequency of each of the first and second filters contribute directly to the overall determination of the effective state.

Adding the occlusion value has been found to be beneficial where despite the presence of effectively fitted protection, sound would appear to be amplified rather than attenuated, as for the case where there is occlusion of the user's own voice as described above. In such a situation the attenuation value could be quite low.

More specifically, when an individual speaks with the hearing protection device fitted, frequencies close to the fundamentals of speech are amplified in the ear canal. The attenuation value may therefore be small on account of the larger amplitude of the filtered internal sound signal. In embodiments where the second filter is designed to pass frequencies around the fundamentals of speech, calculating the occlusion value by dividing the second filtered internal sound signal by the second filtered ambient sound signal would result in a large and positive value when an individual is speaking. Therefore, using the occlusion value in the summation to calculate the effectiveness metric compensates for the smaller attenuation value.

The same principle could be applied to compensate for known behaviour of sound filtered at other frequencies and combinations of frequencies in response to the fitting the wearable hearing protection device.

In embodiments where the hearing protection device comprises a device designed for insertion into an ear canal, the effective state may represent whether the device is present in the ear canal.

The effective state may be binary, i.e. the acoustic barrier of the hearing protection device may be deemed to be effective, or not effective. Alternatively, the effective state may take one of a set of more possible states indicating different levels of efficacy. For example, the wearable hearing protection device could be indicated to be present, but incorrectly fitted, thus ineffectively applying the acoustic barrier. Alternatively, or additionally, the effective state could be that additional hearing protection, e.g. in the form of over-ear defenders, is present.

In a set of embodiments, the combination of two or more comparison values is compared to a threshold in order to determine the effective state of the acoustic barrier.

In a set of embodiments where an effectiveness metric is calculated in order to determine the effective state of the hearing protection device, the effectiveness metric, or average effectiveness metric is compared to one or more threshold values. The one or more threshold values may be used to determine whether the acoustic barrier is effectively applied or not, which may correspond to whether the wearable hearing protection device is present, or not present. In a set of embodiments where the wearable hearing protection device comprises an earpiece designed for insertion into an ear canal, a threshold is used to determine whether the device is "In Ear" or "Out of Ear". Another threshold value may be used to determine whether an additional outer hearing protection device is being used as well as the earpiece designed for insertion into an ear canal. In a set of embodiments, a further threshold value may be used to determine the quality of the fit of the wearable hearing protection device. The outcomes of these comparisons generate an indication of a corresponding effective state of the wearable hearing protection device.

In a set of embodiments, if the ambient sound signal is not above a noise floor of the measuring system, a void wearable hearing protection device status is returned. In such circumstances, there would be no requirement to have the wearable hearing protection device fitted due to the low noise levels. It is therefore not important to determine the effective state of the acoustic barrier.

In a set of embodiments, time averaging is applied to the filtered ambient sound signals and filtered internal sound signals. Alternatively, time averaging may be applied at another stage of the signal processing, e.g to the raw received sound signals, or the effectiveness metric before comparison to the one or more threshold values.

In a set of embodiments, this time averaging is achieved by calculating the mean square of the filtered or raw sound signals, where the square of the instantaneous magnitude of the signal is averaged over a period of time e.g. 5-30s. This may result in a signal that is more stable. Using the mean square can result in large magnitudes of the calculated effectiveness metric, therefore, additionally, an upper limit may be applied to the attenuation value or occlusion value before combination or to the effectiveness metric before comparison to the one or more threshold values.

Advantageously, applying some form of averaging in the process of determining the effective state allows spurious 'noisy' variation in the calculated comparison values and/or effectiveness metric to be discounted. Accordingly, a more reliable and stable estimate of the effective state of the hearing protection device may be determined.

In a set of embodiments, the effective state of the hearing protection device is determined repeatedly during normal use. In a set of embodiments, the effective state of the hearing protection device is determined using continually collected sound data only, i.e. no test sound is required to be played. Advantageously, this allows the effective state of the hearing protection device to be determined frequently, without disturbing the individual using the device. The internal sound signal and ambient sound signal may be received by a processing element in the hearing protection device or external processor for other purposes, as well as determining the effective state of the hearing protection device.

Repeatedly determining the effective state of the hearing protection device during the period over which the individual is using the device can better help to detect, and alert the individual to, a lack of hearing protection. This may for example help to discourage removal of the protection device after a period of time (which the user may have become accustomed to doing), to remind the user to replace it properly if it has been removed legitimately (e.g. in a break or when working in a low noise environment) or to refit and/or adjust the protection device if the device has become displaced over a period of time.

The effective state of the hearing protection device may be determined at regular intervals, e.g, every minute. By determining effective state provided by the hearing device at regular time intervals, compliance can be ensured in a straightforward way and safe behaviour may be reinforced.

The calculations referred to herein could be carried out by the hearing protection device. In a set of embodiments however, data relating to the one or more of the ambient sound signal, internal sound signal, first filtered ambient sound signal, first filtered internal sound signal, second filtered ambient sound signal, second filtered internal sound signal, or any number of filtered sound signals is exported to an external processor such as an external device or a cloud service in order to carry out the calculations.

An indication of the effective state is generated dependent on the comparison to one or more threshold values. The indication may be given as an output to another system, a value stored in memory, or an alert. An alert may be visual, audible or haptic, and may be provided to the user of the wearable hearing protection device, or another individual, e.g. a supervisor. If the indication is output into another system, the other system could be a separate device, connected to the hearing protection device through wired or wireless connection. The separate device could be a mobile phone or radio device, a device for storing the hearing protection. Alternatively, it may be an external server with cloud storage facilities. Any combination of the above indications of the effective state of the hearing protection device could be given.

The invention could be implemented by determining the effective state of an acoustic barrier in relation to just one of the user's ears. This might be sufficient where the acoustic barrier is provided by a pair of over-ear hearing protectors which tend to be worn or taken off from both ears together. In a set of embodiments however the effective state of an acoustic barrier in respect of each ear is determined independently in accordance with the invention set out above. Separate indications of effective may therefore be provided or they could be combined - e.g. to indicate compliance if both ears have an acceptable acoustic barrier but non-compliance if either or both of them do not.

Features of any aspect or embodiment described herein may, wherever appropriate, be applied to any other aspect or embodiment described herein. Where reference is made to different embodiments or sets of embodiments, it should be understood that these are not necessarily distinct but may overlap.

BRIEF DESCRIPTION OF THE DRAWINGS

Certain preferred embodiments of the invention will now be described, by way of example only, with reference to the accompanying drawings, in which:

Figure 1 shows a schematic cross-section of an earplug forming part of a wearable hearing protection device in accordance with an embodiment of the present invention;

Figures 2A and 2B are schematic diagrams of a wearable hearing protection device in accordance with the embodiment of the present invention shown in Figure 1;

Figure 3A is a simplified schematic graph showing a band-pass filter which may be used in accordance with a first set of embodiments of the present invention;

Figure 3B is a simplified schematic graph showing another band-pass filter which may be used in accordance with a first set of embodiments of the present invention;

Figure 4 is a plot showing processed sound data being used to return an effective state in accordance with a first set of embodiments of the present invention; and

Figure 5 is a flow diagram showing the comparison of processed sound data to one or more threshold values in accordance with embodiments of the present invention and

Figure 6 is a simplified schematic graph showing a set of filters which may be used in accordance with a second set of embodiments of the present invention; and

Figure 7 is a flow diagram showing the processing steps applied to sound data being used to return an effective state in accordance with a first set of embodiments of the present invention.

DETAILED DESCRIPTION

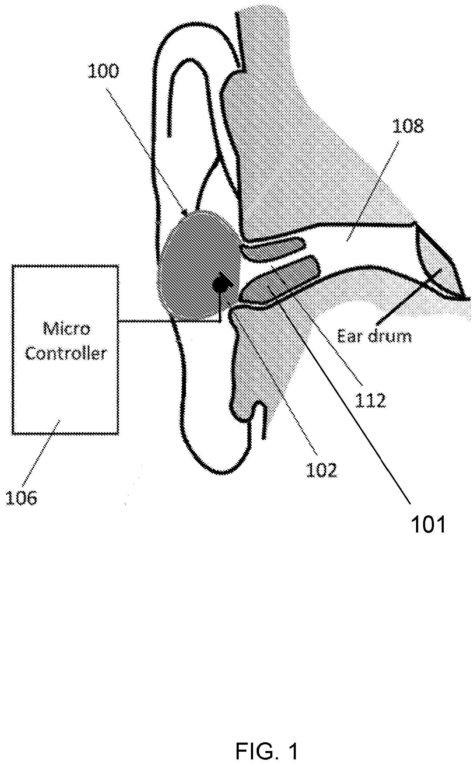

Figure 1 shows a schematic cross-section of an earplug 100, forming part of an in-ear hearing protection device in accordance with the invention, a portion 101 of which is inserted into the ear canal 108 of an individual. Therefore, the earplug 100 provides an acoustic barrier which attenuates sounds from the local environment (e.g. outside of the ear) so that the ear drum of the individual is subjected to reduced amplitude sounds. Whilst a singular earplug 100 is shown in Figure 1, the individual is likely to use earplugs in both of their ears to provide protection against damage to their hearing.

The position of the earplug 100 shown in Figure 1 is the correctly fitted position. In this configuration, the insertion portion 101 is fully inserted so that it creates a seal within the ear canal 108. There are no air pockets between the earplug portion 101 and the walls of the ear canal 108. In this position the level of attenuation provided by the earplug 100 is approximately the intended level of attenuation that can be provided by the earplug 100. In this correctly fitted position, the optimum protection against damage is provided.

The earplug 100 further comprises a microphone 102, microcontroller 106 and a channel 112 in the insertion portion 101 through which sound can pass into the ear canal 108 and which is in communication with the microphone 102. The microphone 102 therefore detects sound in the ear canal 108, behind the acoustic barrier provided by the earplug 100.

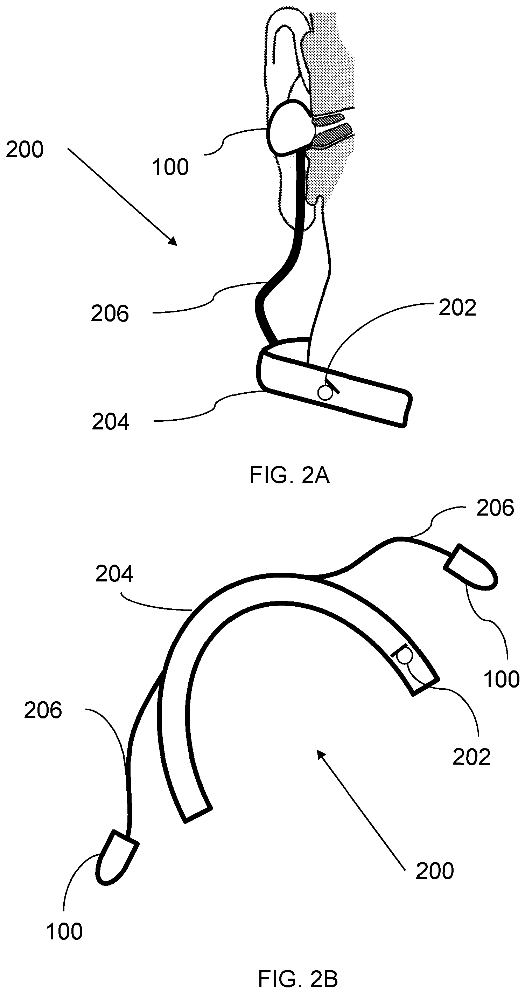

Figure 2A shows a hearing protection device 200 which comprises the earplug component 100 as shown in Figure 1, and additionally comprises an external microphone 202 on a wearable collar 204, connected to the earplug component 100 by connecting wire 206.

Figure 2B shows a configuration where the hearing protection device 200 comprises two ear plug components 100 attached to wearable collar 204 via connecting wires 206, in order to provide protection for use in both ears.

The external microphone 202 measures the sound outside of the earplug 100, i.e. in the local environment outside of the earplug 100. The external microphone 202 can therefore be considered to measure the ambient sound that the ear of an individual would be subjected if the earplug 100 was not fitted.

In Figure 2A, the external microphone 202 is shown as being located on a wearable collar which is connected to, but physically separated from the earplug component 100. In other embodiments not shown, the external microphone could be provided separately e.g. in a mobile device. Alternatively, and also not shown, the external microphone could be located on the structure of the earplug 100, but with a channel connecting it to the outside of the acoustic barrier

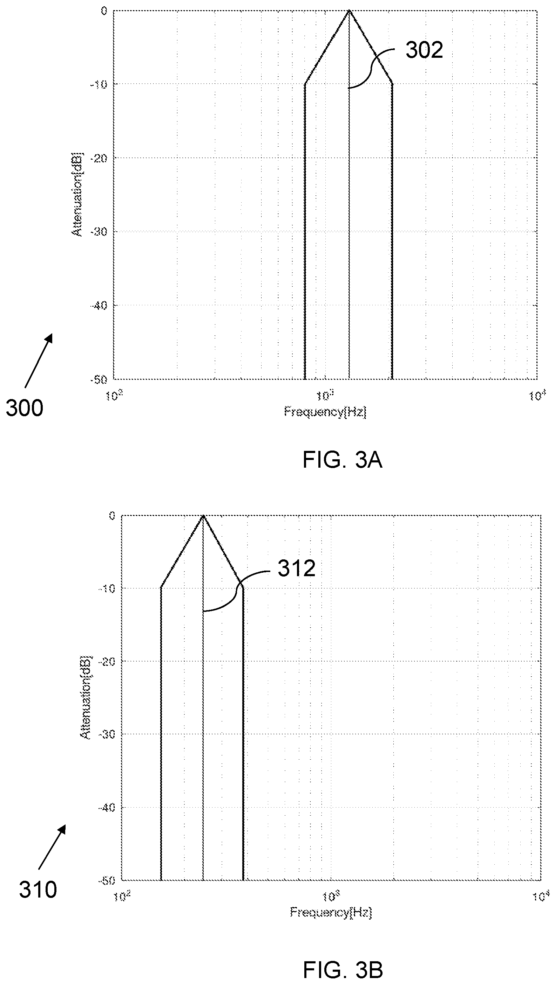

In use sound signals measured by these microphones 102, 202 are received and passed to the microcontroller 106 to be processed. The processing applied to the signals in a first set of embodiments is described in greater detail below with reference to Figs. 3A and 3B.

Figure 3A shows a simplified representation of the frequency response of a first band pass filter 300 which is applied both to the sound signal received from the microphone 102 behind the acoustic barrier, and to the sound signal from the microphone 202 outside of the acoustic barrier.

The band pass filter 300 passes frequencies around a centre frequency 302 of approximately 800 - 1200 Hz. This frequency has been found to be significant when the earplug 100 is properly fitted. Analysing sound from this frequency range in isolation thus gives a good indication as to the presence of the earplug 100 in the ear canal 100.

The band pass filter 300 can in the form of a BiQuad bell curve, the properties of which can be controlled by varying the centre frequency 302, gain, and Q-value, which defines the filter bandwidth. However other filter types could be used. For analysing attenuation, an effective centre frequency 302 has been found to be in the range 800-1200Hz.

The microcontroller 106 calculates an attenuation value in the form of an 'ambient attenuation ratio' by dividing the mean-squared value of the filtered signal representing sound from outside the ear after application of the filter 300 by the mean-squared value of the signal representing filtered sound from inside the ear. The ambient attenuation ratio $AR_A$ is given below with F denoting the filtered sound signals, and subscript A denoting the frequency band used to measure attenuation.

$$AR_A = \frac{F_{A,ambient}}{F_{A,earpiece}}$$

This ambient attenuation ratio is large and positive when an individual has fitted the earplug 100 correctly, and noise levels in the environment are high.

The applicant has found the resulting attenuation ratio is affected if the individual is speaking whilst the earplug 100 is fitted. This is due to the occlusion effect, which amplifies frequencies close to the fundamentals of speech in the ear canal. When an individual speaks with the hearing protection device fitted, the ambient attenuation value $AR_A$ defined above would become smaller on account of the larger amplitude of the $F_{A,earpiece}$ signal. The hearing protection device may by consequence appear to amplify, rather than attenuate, environmental noise. This is the case especially if the noise levels in the environment are low, and could erroneously cause the above method to determine that the hearing protection device is incorrectly fitted, or not fitted at all.

To address this problem, the microcontroller applies a further band pass filter 310 which can, for example only, be in the form of a BiQuad bell curve with a centre frequency 312 close to the fundamentals of speech to both of the received sound signals form the inner and outer microphones 102, 202 respectively, as shown in Figure 3B. The further filter 310 filters the signal to select a frequency range with a lower centre frequency 312 than the first applied band pass filter 300 (e.g. 150-500Hz).

For the resulting filtered ambient and internal signals, the microcontroller 106 calculates a 'occlusion value' using the inverse ratio compared to that used for the ambient attenuation ratio - i.e. with the sound signal from the microphone 102 from inside the barrier divided by the magnitude of the filtered sound from signal from the microphone 202 outside the barrier. The speech attenuation ratio $AR_S$ is given below with F denoting the filtered sound signals, and subscript S denoting the frequency band to measure the occlusion effect from speech.

$$AR_S = \frac{F_{S,earpiece}}{F_{S,ambient}}$$

This occlusion value becomes large and positive when the individual has fitted the ear piece, and is producing sound through speech. A better fitted earpiece will result in a greater amplification of speech in the ear canal. Therefore, as for $AR_A$, $AR_S$ value indicates better attenuation.

The occlusion value is combined with the ambient attenuation ratio to reach an effectiveness metric T. This could be through simple summation as shown below, or a weighted sum.

$$T = AR_A + AR_S$$

Combining the attenuation ratio and the occlusion value in such a way means that a large value of T indicates effectiveness of the sound barrier which should provide good attenuation of environmental noise, and by consequence effective use of the wearable hearing protection device.

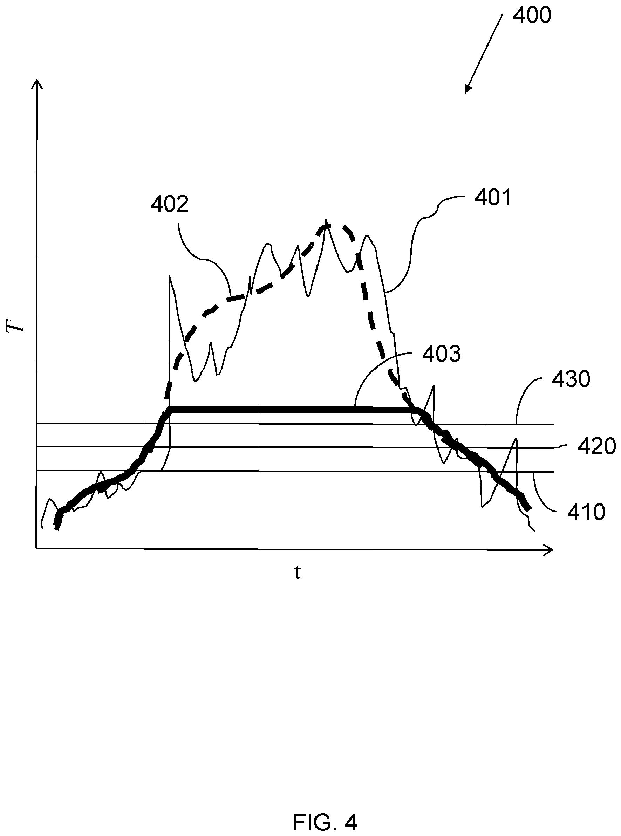

Figure 4 shows the effectiveness metric T 401 calculated over time t, as well as an exemplary set of threshold criteria 410, 420, 430. It has been appreciated by the applicant that the instantaneous effectiveness metric 401 is too unstable to be used for meaningful output. Data processing of the effectiveness metric may be improved by using one or more different types time averaging, to produce a smoother output as shown by the curve 402.

An upper limit can be applied to the effectiveness metric before averaging, whilst maintaining a range that results in a sufficient variation in magnitude to distinguish significant attenuation from lower levels of attenuation, or lack of attenuation altogether.

Signal 403 is a time-averaged and magnitude-limited effectiveness metric as generated from the raw effectiveness metric 401 for the received sound signals over time. This can produce an output more suitable for the purpose of identifying distinct changes in applied hearing protection.

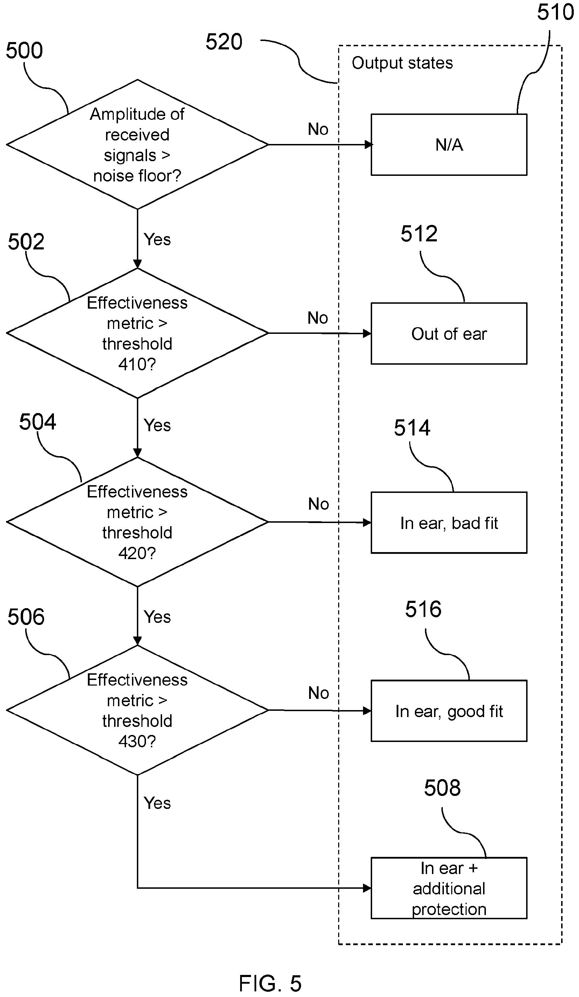

Figure 5 is a flow chart showing an exemplary method for applying the applied threshold values and corresponding output states 520 indicating the effective state of the hearing protection device. First, if the environmental sound is not above the noise floor of the measurement system, sound levels are too low to perform the ratio calculations as explained above. In step 500, if the magnitude of the environmental noise is too low, a void hearing protection device status 510 is returned.

If sound levels have been determined to be sufficiently high in step 500, the processed effectiveness metric 403 can then be compared against a set of threshold criteria. If the effectiveness metric is below the lower threshold 410, the hearing protection device 200 is deemed to have a state 512 of "Out of ear". If the effectiveness metric is above the lower threshold 410, the hearing protection device 200 is deemed to have an "In ear" state. If the effectiveness metric is above the lower threshold 410, but below the intermediate threshold 420, the hearing protection device is deemed to be "In Ear" but with a poor quality fit as depicted by state 514. If the effectiveness metric is above the intermediate threshold 420, but below the upper threshold 430, the output state 516 indicates the device is "In ear" with a good quality fit, and if the effectiveness metric is above the upper threshold 430, the hearing protection device is deemed to be "In ear" with an additional outer hearing protection device also applied as labelled in state 508.

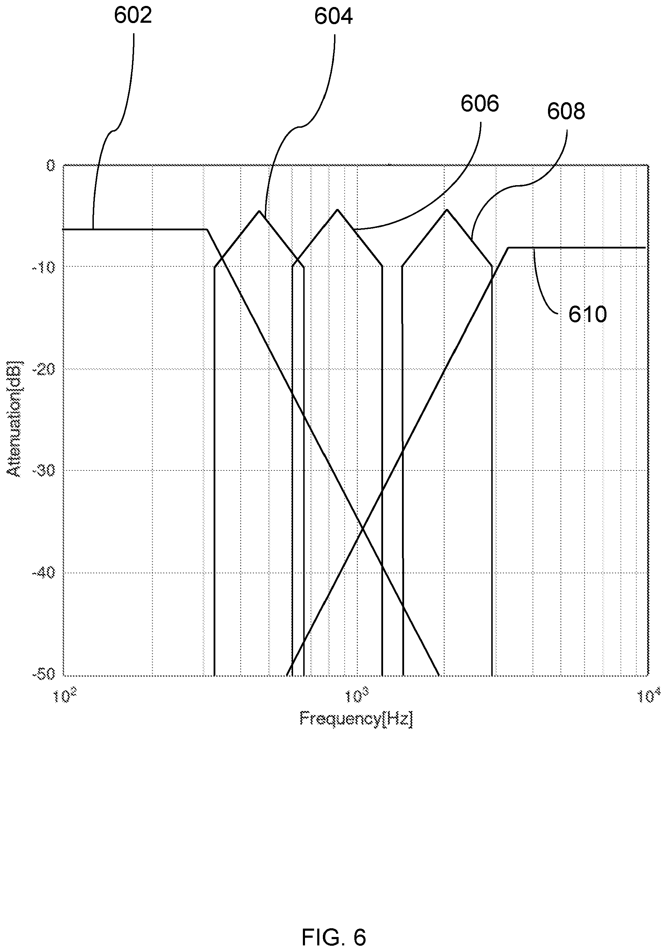

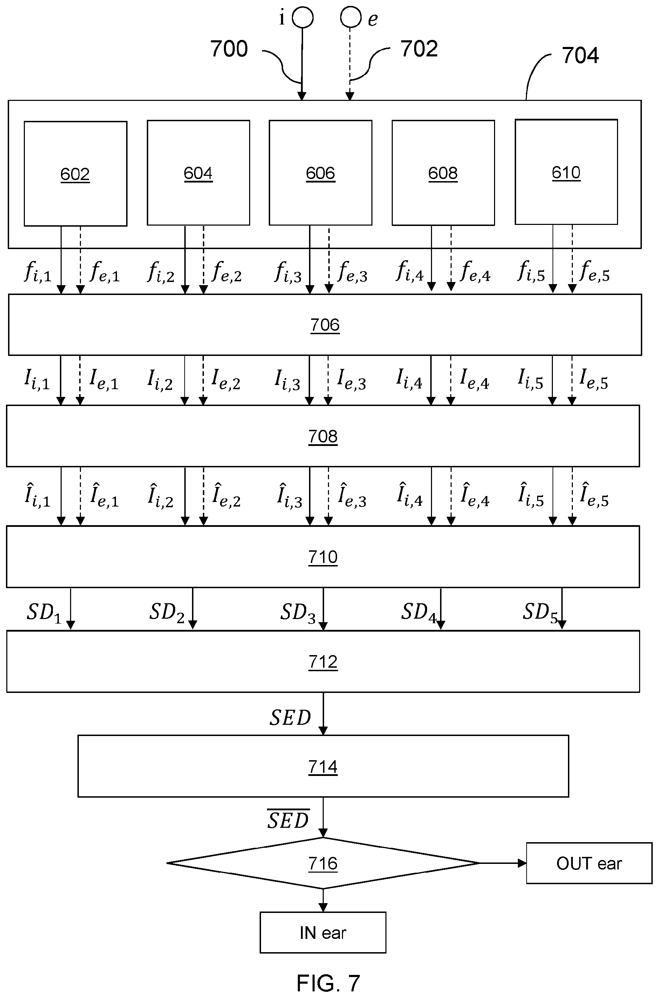

Figures 6 and 7 show the processing applied to the ambient and internal sound signals in a second set of embodiments, where more than two filters are used. Although any number of filters may be used, in the second set of embodiments described herein, five different filters are used.

Figure 6 shows a simplified representation of the frequency response for each of these five different filters. Filter 602 is a low-pass filter, filters 604, 606 and 608 are band-pass filters, and filter 610 is a high-pass filter. Different weights can be applied to each respective filter, e.g. to give a flatter overall response.

Each filter is applied both to the sound signal received from the microphone 102 behind the acoustic barrier, and to the sound signal from the microphone 202 outside of the acoustic barrier. The Applicant has appreciated that the overall shape of the frequency response differs in dependence on whether the hearing protection device is fitted in the ear or not.

Figure 7 is a flow chart showing an exemplary method for determining an effective state of the hearing protection device in accordance with the second set of embodiments. In step 704, the internal sound signal 700 received from the microphone 102 behind the acoustic barrier (i), and the external sound signal 702 received from the microphone 202 outside the acoustic barrier (e), are given as input to each of the filters 602, 604, 606, 608 and 610.

In step 706, for each filtered sound signal $f_{i,n}$, $f_{e,n}$, $I_{i,n}$, $I_{e,n}$ is calculated which represents the sound level in the corresponding frequency band.

In step 708, each the intensity values $I_{i,n}$, $I_{e,n}$ are normalised by an intensity value at one selected normalisation frequency. For example, the filtered signals may be normalised by the intensity value $I_{i,3}$, $I_{e,3}$ at the centre frequency of the middle band-pass filter 606.

$$\hat{I}_{i,n} \;=\; \frac{I_{i,n}}{|I_{i,3}|} \qquad \hat{I}_{e,n} \;=\; \frac{I_{e,n}}{|I_{e,3}|}$$

Normalising each of the intensity values by the magnitude of the intensity value of the middle band-pass filter 606 means that both the normalised internal intensity value and normalised external intensity value at the middle frequency of $f_{i,3}$ will have the same value, i.e., 1 (on account of dividing the magnitude of the intensity value by itself). As a result, the internal frequency response and the ambient frequency response will intersect at the middle frequency. Although the middle band-pass filter frequency is used here, other selected frequencies can also be used. The Applicant has found that this normalisation step may be useful for analysing the difference in shape of the frequency responses, effectively overlapping the two frequency responses such that the difference in shape is more straightforward to calculate because any systematic underlying level difference is removed. However, this normalisation step is not essential for performing the analysis.

In step 710, for each frequency band, a squared difference is used to calculate a comparison value between the normalised internal and ambient intensity values. For example, for a frequency band number n, the squared difference is calculated as follows:

$$SD_n \;=\; (\hat{I}_{e,n} - \hat{I}_{i,n})^2$$

In step 712, the Squared Euclidean Difference (*SED*) between the filtered internal and external signals is calculated for each frequency band as shown by the equation below.

$$SED = \sum_{n=1}^{5} SD_n$$

The *SED* provides an effectiveness metric by which the effective state of the hearing protection device can be measured. A large *SED* indicates that there is a large difference between the shape of the internal frequency response and the external frequency responses, and accordingly indicates that the hearing protection device is effective, e.g. that it is "In-Ear".

For the frequency band n which was used for normalisation in step 708, (e.g. the middle band with index 3, as explained above), both the normalised internal intensity value and normalised external intensity value will have unitary magnitude, therefore, the squared difference will be equal to 0. Accordingly, the difference in the magnitude of the intensity values at the middle frequency band does not contribute to the total of the squared Euclidean difference, rather the purpose of the middle band is to provide a normalisation value, which is used to compare the shape of the responses, as described above.

Steps 704 to 712 are typically carried out every second, but the following steps of 714 and 716 are typically be completed every minute. This allows the any spurious 'noisy' variation in the results of the comparisons carried about in step 710 to be averaged out. In step 714, $\overline{SED}$, the mean of a set of sixty *SED* values calculated across a minute is calculated. However, other methods could be used to calculate the average $\overline{SED}$, including the median of the sixty *SED* values.

In step 716, *SED* is compared to a threshold, and the effective state of the hearing protection device is assumed to be "In Ear" if the $\overline{SED}$ is above the threshold. The earplug is assumed to be "Out of Ear" if $\overline{SED}$ is below the threshold. Of course, it may be possible to apply a number of different thresholds, as described with reference to Figure 5, for example.

Alternatively, instead of calculating an average *SED* and comparing the average value to a threshold every minute, the *SED* could be compared to a threshold every second, and the effective state of the hearing protection device can be determined in dependence on the number of times the *SED* exceeds the threshold across a minute. The outcome of the threshold comparison shown in Figures 4 and 5, or Figure 7, is then used to generate an indication of the effective state of the hearing protection device. This indication could be an output to another system, which collects the data for retrospective review after the period of noise exposure, or could be output to be stored on the device itself. The provision of an indication of the effective state of the hearing protection device facilitates an ability to review noise exposure retrospectively. This capability may be useful in the context of monitoring compliance in a workforce. It can allow for targeted and appropriate intervention based on the usage patterns of the hearing protection device for an individual, and the noise levels they've been exposed to. Retrospective review could be completed regularly, for instance, at the end of a working day, or it could be completed as part of a compliance audit.

The indication could also be used to inform an alert provided to the user of the hearing protection device. For example, this could be an audible alarm if noise levels in the environment are high, but the ear plugs are deemed not to be worn. This may help to provide immediate prompts targeted at reducing the potential for hearing loss.

It will be appreciated by those skilled in the art that the invention has been illustrated by describing one or more specific embodiments thereof, but is not limited to these embodiments; many variations and modifications are possible, within the scope of the accompanying claims.

**Claims**

1. A method of determining an effective state of an acoustic barrier of a hearing protection device (100; 200) comprising:

   receiving an ambient sound signal (702) representative of sound outside of the acoustic barrier;
   receiving an internal sound signal (700) representative of sound behind the acoustic barrier;
   processing (704) each of the ambient sound signal (702) and the internal sound signal (700) using a filter (602) with a first frequency characteristic to generate:

      a first filtered ambient sound signal; and,
      a first filtered internal sound signal; and,

   using a second filter (604) with a second frequency characteristic to generate:

      a second filtered ambient sound signal; and,
      a second filtered internal sound signal;

   **characterised by**:

      processing (704) each of the internal sound signal (700) and the ambient sound signal (702) using one or more further filters (606, 608, 610) to generate respective filtered internal and ambient sound signals;
      calculating (706) a respective intensity value for each of the filtered internal and ambient sound signals to generate a set of internal intensity values and a set of ambient intensity values for the corresponding received internal sound signal (700) and received ambient sound signal (702) respectively; wherein each intensity value is representative of the intensity of the sound in a frequency band isolated by the corresponding filter (602, 604, 606, 608, 610);
      normalising (708) each of the intensity values in the set of internal intensity values by an internal intensity value at a selected normalisation frequency to generate a normalised set of internal intensity values;
      normalising (708) each of the intensity values in the set of ambient intensity values by an ambient intensity value at the same selected normalisation frequency, to generate a normalised set of ambient intensity values;
      comparing (716) the shape of the frequency response of the internal sound signal (700) and the shape of the frequency response of the ambient sound signal (702) using the normalised set of internal intensity values and the normalised set of ambient intensity values;
      determining the effective state (520) of the acoustic barrier using the comparison of the shapes of the frequency responses of the internal sound signal (700) and the ambient sound signal (702); and,
      generating an indication of said effective state (520).

2. The method of claim 1, wherein applying each filter (602, 604, 606, 608, 610) to a received sound signal (700, 702) generates a respective filtered sound signal which is isolated for a corresponding frequency band, wherein the frequency band is at least in part determined by a corresponding centre frequency or cut-off frequency of the filter.

3. The method of claim 1 or 2, wherein the selected normalisation frequency corresponds to a centre frequency of the frequency characteristic of one of the filters (602, 604, 606, 608, 610).

4. The method of any preceding claim, comprising generating (710) a set of comparison values, wherein each comparison value in the set corresponds to a respective frequency band, and each comparison value is calculated by comparing the corresponding ambient intensity value or normalised ambient intensity value and the internal intensity value or normalised internal intensity value for the corresponding frequency band.

5. The method of claim 4, wherein each comparison value is calculated as the squared difference between the ambient intensity value or normalised ambient intensity value and the internal intensity value or normalised internal intensity value for the corresponding frequency band.

6. The method of claim 4 or 5, comprising combining (712) two or more comparison values to calculate an effectiveness metric for each set of comparison values that are generated, and using the effectiveness metric to determine the effective state (520) of the acoustic barrier.

7. The method of claim 6, comprising, for a set of effectiveness metrics calculated over a period of time:

    comparing (502, 504, 506) each effectiveness metric in the set of effectiveness metrics to a threshold value (410, 420, 430); and determining the effective state (520) in dependence on the number of instances that the effectiveness metric exceeds the threshold value (410, 420, 430) in the set of effectiveness metrics.

8. The method of claim 6 or 7, comprising, for a set of effectiveness metrics calculated over a period of time:

    calculating (714) an average effectiveness metric for the set of effectiveness metrics; and

comparing (716) the average effectiveness metric to a threshold value (410, 420, 430) to determine the effective state (520).

9. The method of any of claims 6 to 8, comprising determining the effective state (520) of the acoustic barrier by comparing (502, 504, 506) the effectiveness metric or average effectiveness metric to a plurality of threshold values (410, 420, 430).

10. The method of any preceding claim, wherein the effective state (520) comprises whether the acoustic barrier is in place and effective (508, 516), or is either not in place (512) or not effective (514).

11. The method of any preceding claim, wherein the effective state (520) represents whether the acoustic barrier is fitted within an ear canal (508, 514, 516).

12. The method of any preceding claim, wherein the effective state (520) represents whether an additional external acoustic barrier is fitted (508) .

13. The method of any preceding claim comprising comparing (500) the ambient sound signal (702) to a minimum threshold and indicating a void effective state (510) if the magnitude of the ambient sound signal (702) is below said minimum threshold.

14. The method of any preceding claim, wherein the effective state (520) is determined repeatedly during normal use.

15. The method of any preceding claim, wherein the effective state (520) is determined using continually collected sound data, without playing a test sound.

**Patentansprüche**

1. Verfahren zur Bestimmung des Wirkungszustands einer akustischen Barriere einer Gehörschutzvorrichtung (100; 200), umfassend:

    Empfangen eines Umgebungsschallsignals (702), das für Schall außerhalb der akustischen Barriere repräsentativ ist; Empfangen eines internen Schallsignals (700), das für Schall hinter der akustischen Barriere repräsentativ ist; Verarbeiten (704) des Umgebungsschallsignals (702) und des internen Schallsignals (700) unter Verwendung eines Filters (602) mit einer ersten Frequenzcharakteristik, um Folgendes zu erzeugen:

        ein erstes gefiltertes Umgebungsschallsignal; und,

ein erstes gefiltertes internes Schallsignal; und,

Verwenden eines zweiten Filters (604) mit einer zweiten Frequenzcharakteristik, um Folgendes zu erzeugen:

ein zweites gefiltertes Umgebungsschallsignal; und,
ein zweites gefiltertes internes Schallsignal;

**gekennzeichnet durch**:

Verarbeiten (704) des internen Schallsignals (700) und des Umgebungsschallsignals (702) unter Verwendung eines oder mehrerer weiterer Filter (606, 608, 610), um jeweils gefilterte interne und Umgebungsschallsignale zu erzeugen;
Berechnen (706) eines jeweiligen Intensitätswerts für jedes der gefilterten internen und Umgebungsschallsignale, um einen Satz von internen Intensitätswerten und einen Satz von Umgebungsintensitätswerten für das entsprechende empfangene interne Schallsignal (700) und das empfangene Umgebungsschallsignal (702) zu erzeugen; wobei jeder Intensitätswert für die Intensität des Schalls in einem durch den entsprechenden Filter (602, 604, 606, 608, 610) isolierten Frequenzband repräsentativ ist;
Normalisieren (708) jedes der Intensitätswerte in dem Satz von internen Intensitätswerten durch einen internen Intensitätswert bei einer ausgewählten Normalisierungsfrequenz, um einen normierten Satz von internen Intensitätswerten zu erzeugen;
Normalisieren (708) jedes der Intensitätswerte in dem Satz von Umgebungsintensitätswerten durch einen Umgebungsintensitätswert bei derselben ausgewählten Normalisierungsfrequenz, um einen normierten Satz von Umgebungsintensitätswerten zu erzeugen;
Vergleichen (716) der Form der Frequenzantwort des internen Schallsignals (700) mit der Form der Frequenzantwort des Umgebungsschallsignals (702) unter Verwendung des normierten Satzes von internen Intensitätswerten und des normierten Satzes von Umgebungsintensitätswerten;
Bestimmen des effektiven Zustands (520) der akustischen Barriere unter Verwendung des Vergleichs der Formen der Frequenzantwort des internen Schallsignals (700) und des Umgebungsschallsignals (702); und Erzeugen einer Anzeige des genannten effektiven Zustands (520).

2. Verfahren nach Anspruch 1, wobei das Anwenden jedes Filters (602, 604, 606, 608, 610) auf ein empfangenes Schallsignal (700, 702) ein jeweiliges gefiltertes Schallsignal erzeugt, das für ein entsprechendes Frequenzband isoliert ist, wobei das Frequenzband zumindest teilweise durch eine entsprechende Mittenfrequenz oder Grenzfrequenz des Filters bestimmt wird.

3. Verfahren nach Anspruch 1 oder 2, wobei die ausgewählte Normalisierungsfrequenz einer Mittenfrequenz der Frequenzcharakteristik eines der Filter (602, 604, 606, 608, 610) entspricht.

4. Verfahren nach einem vorstehenden Anspruch, umfassend das Erzeugen (710) eines Satzes von Vergleichswerten, wobei jeder Vergleichswert in dem Satz einem jeweiligen Frequenzband entspricht und jeder Vergleichswert durch Vergleichen des entsprechenden Umgebungsintensitätswerts oder normierten Umgebungsintensitätswerts mit dem internen Intensitätswert oder normierten internen Intensitätswert für das entsprechende Frequenzband berechnet wird.

5. Verfahren nach Anspruch 4, wobei jeder Vergleichswert als die quadrierte Differenz zwischen dem Umgebungsintensitätswert oder dem normierten Umgebungsintensitätswert und dem internen Intensitätswert oder dem normierten internen Intensitätswert für das entsprechende Frequenzband berechnet wird.

6. Verfahren nach Anspruch 4 oder 5, umfassend das Kombinieren (712) von zwei oder mehr Vergleichswerten zur Berechnung einer Wirksamkeitskennzahl für jeden erzeugten Satz von Vergleichswerten und das Verwenden der Wirksamkeitskennzahl zur Bestimmung des Wirksamkeitszustands (520) der akustischen Barriere.

7. Verfahren nach Anspruch 6, umfassend, für einen Satz von Wirksamkeitskennzahlen, die über einen Zeitraum berechnet werden:

Vergleichen (502, 504, 506) jeder Wirksamkeitskennzahl in dem Satz von Wirksamkeitskennzahlen mit einem Schwellenwert (410, 420, 430); und
Bestimmen des Wirksamkeitszustands (520) in Abhängigkeit von der Anzahl der Fälle, in denen die Wirksamkeitsmetrik den Schwellenwert (410, 420, 430) in dem Satz von Wirksamkeitsmetriken überschreitet.

8. Verfahren nach Anspruch 6 oder 7, umfassend, für einen Satz von Wirksamkeitskennzahlen, die über einen Zeitraum berechnet werden:

Berechnen (714) einer durchschnittlichen Wirksamkeitskennzahl für den Satz von Wirksamkeitskennzahlen; und

Vergleichen (716) der durchschnittlichen Wirksamkeitskennzahl mit einem Schwellenwert (410, 420, 430), um den Wirksamkeitszustand (520) zu bestimmen.

9. Verfahren nach einem der Ansprüche 6 bis 8, umfassend das Bestimmen des Wirksamkeitszustands (520) der akustischen Barriere durch Vergleich (502, 504, 506) der Wirksamkeitskennzahl oder der durchschnittlichen Wirksamkeitskennzahl mit einer Vielzahl von Schwellenwerten (410, 420, 430).

10. Verfahren nach einem vorstehenden Anspruch, wobei der Wirksamkeitszustand (520) umfasst, ob die akustische Barriere vorhanden und wirksam ist (508, 516) oder entweder nicht vorhanden (512) oder nicht wirksam (514) ist.

11. Verfahren nach einem vorstehenden Anspruch, wobei der Wirksamkeitszustand (520) angibt, ob die akustische Barriere in einem Gehörgang (508, 514, 516) sitzt.

12. Verfahren nach einem vorstehenden Anspruch, wobei der Wirksamkeitszustand (520) angibt, ob eine zusätzliche externe akustische Barriere angebracht ist (508).

13. Verfahren nach einem der vorstehenden Ansprüche, umfassend das Vergleichen (500) des Umgebungsschallsignals (702) mit einem Mindestschwellenwert und Anzeigen eines leeren Wirksamkeitszustands (510), wenn die Amplitude des Umgebungsschallsignals (702) unterhalb des Mindestschwellenwerts liegt.

14. Verfahren nach einem vorstehenden Anspruch, wobei der Wirksamkeitszustand (520) während des normalen Betriebs wiederholt bestimmt wird.

15. Verfahren nach einem vorstehenden Anspruch, wobei der Wirksamkeitszustand (520) unter Verwendung kontinuierlich erfasster Schalldaten bestimmt wird, ohne einen Testschall wiederzugeben.

## Revendications

1. Procédé de détermination d'un état effectif d'une barrière acoustique d'un dispositif de protection auditive (100 ; 200) comprenant :

la réception d'un signal sonore ambiant (702) représentatif d'un son situé à l'extérieur de la barrière acoustique ;
la réception d'un signal sonore interne (700) représentatif du son situé derrière la barrière acoustique ;
le traitement (704) de chacun parmi le signal sonore ambiant (702) et le signal sonore interne (700) en utilisant un filtre (602) ayant une première caractéristique de fréquence pour générer :

un premier signal sonore ambiant filtré ; et,
un premier signal sonore interne filtré ; et,

l'utilisation d'un deuxième filtre (604) ayant une deuxième caractéristique de fréquence pour générer :

un deuxième signal sonore ambiant filtré ; et,
un deuxième signal sonore interne filtré ;

**caractérisé par** :

le traitement (704) de chacun parmi le signal sonore interne (700) et le signal sonore ambiant (702) en utilisant un ou plusieurs filtres (606, 608, 610) supplémentaires pour générer des signaux sonores interne et ambiant filtrés respectifs ;
le calcul (706) d'une valeur d'intensité respective pour chacun parmi les signaux sonores interne et ambiant filtrés pour générer un ensemble de valeurs d'intensité interne et un ensemble de valeurs d'intensité ambiante pour le signal sonore interne (700) reçu et le signal sonore ambiant (702) reçu correspondants respectivement ; dans lequel chaque valeur d'intensité est représentative de l'intensité du son dans une bande de fréquences isolée par le filtre (602, 604, 606, 608, 610) correspondant ;
la normalisation (708) de chacune parmi les valeurs d'intensité dans l'ensemble de valeurs d'intensité interne par une valeur d'intensité interne à une fréquence de normalisation sélectionnée pour générer un ensemble normalisé de valeurs d'intensité interne ;
la normalisation (708) de chacune parmi les valeurs d'intensité dans l'ensemble de valeurs d'intensité ambiante par une valeur d'intensité ambiante à la même fréquence de normalisation sélectionnée, pour générer un ensemble normalisé de valeurs d'intensité ambiante ;
la comparaison (716) de la forme de la réponse en fréquence du signal sonore interne (700) et de la forme de la réponse en fréquence du signal sonore ambiant (702) en utilisant l'ensemble normalisé de valeurs d'intensité interne et l'ensemble normalisé de valeurs d'intensité ambiante ;

la détermination de l'état effectif (520) de la barrière acoustique en utilisant la comparaison des formes des réponses en fréquence du signal sonore interne (700) et du signal sonore ambiant (702) ; et, la génération d'une indication dudit état effectif (520).

2. Procédé selon la revendication 1, dans lequel l'application de chaque filtre (602, 604, 606, 608, 610) à un signal sonore (700, 702) reçu génère un signal sonore filtré respectif qui est isolé pour une bande de fréquences correspondante, dans lequel la bande de fréquences est au moins en partie déterminée par une fréquence centrale ou une fréquence de coupure correspondante du filtre.

3. Procédé selon la revendication 1 ou la revendication 2, dans lequel la fréquence de normalisation sélectionnée correspond à une fréquence centrale de la caractéristique de fréquence d'un des filtres (602, 604, 606, 608, 610).

4. Procédé selon une quelconque revendication précédente, comprenant la génération (710) d'un ensemble de valeurs de comparaison, dans lequel chaque valeur de comparaison dans l'ensemble correspond à une bande de fréquences respective, et chaque valeur de comparaison est calculée en comparant la valeur d'intensité ambiante ou la valeur d'intensité ambiante normalisée correspondante et la valeur d'intensité interne ou la valeur d'intensité interne normalisée pour la bande de fréquences correspondante.

5. Procédé selon la revendication 4, dans lequel chaque valeur de comparaison est calculée comme la différence au carré entre la valeur d'intensité ambiante ou la valeur d'intensité ambiante normalisée et la valeur d'intensité interne ou la valeur d'intensité interne normalisée pour la bande de fréquences correspondante.

6. Procédé selon la revendication 4 ou la revendication 5, comprenant la combinaison (712) de deux valeurs de comparaison ou plus pour calculer une métrique d'efficacité pour chaque ensemble de valeurs de comparaison qui sont généré, et l'utilisation de la métrique d'efficacité pour déterminer l'état effectif (520) de la barrière acoustique.

7. Procédé selon la revendication 6, comprenant, pour un ensemble de métriques d'efficacité calculées sur une période de temps :

la comparaison (502, 504, 506) de chaque métrique d'efficacité dans l'ensemble de métriques d'efficacité à une valeur seuil (410, 420, 430) ; et la détermination de l'état effectif (520) en fonction du nombre d'occurrences où la métrique d'efficacité dépasse la valeur seuil (410, 420, 430) dans l'ensemble de métriques d'efficacité.

8. Procédé selon la revendication 6 ou la revendication 7, comprenant, pour un ensemble de métriques d'efficacité calculées sur une période de temps :

le calcul (714) d'une métrique d'efficacité moyenne pour l'ensemble de métriques d'efficacité ; et la comparaison (716) de la métrique d'efficacité moyenne à une valeur seuil (410, 420, 430) pour déterminer l'état effectif (520).

9. Procédé selon l'une quelconque des revendications 6 à 8, comprenant la détermination de l'état effectif (520) de la barrière acoustique en comparant (502, 504, 506) la métrique d'efficacité ou la métrique d'efficacité moyenne à une pluralité de valeurs seuils (410, 420, 430).

10. Procédé selon une quelconque revendication précédente, dans lequel l'état effectif (520) comprend si la barrière acoustique est en place et efficace (508, 516), ou si elle n'est pas en place (512) ou non efficace (514).

11. Procédé selon une quelconque revendication précédente, dans lequel l'état effectif (520) représente si la barrière acoustique est installée à l'intérieur d'un conduit auditif (508, 514, 516).

12. Procédé selon une quelconque revendication précédente, dans lequel l'état effectif (520) représente si une barrière acoustique externe supplémentaire est installée (508).

13. Procédé selon une quelconque revendication précédente comprenant la comparaison (500) du signal sonore ambiant (702) à un seuil minimal et l'indication d'un état effectif invalide (510) si l'amplitude du signal sonore ambiant (702) est inférieure audit seuil minimal.

14. Procédé selon une quelconque revendication précédente, dans lequel l'état effectif (520) est déterminé de façon répétée pendant une utilisation normale.

15. Procédé selon une quelconque revendication précédente, dans lequel l'état effectif (520) est déterminé en utilisant des données sonores collectées en continu, sans émission d'un son d'essai.

FIG. 1

FIG. 2A

FIG. 2B

FIG. 3A

FIG. 3B

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210241747 A1